# EUROPÄISCHE PATENTANMELDUNG

(11) **EP 1 586 327 A1**
(43) Veröffentlichungstag der Anmeldung: **19.10.2005**
(21) Anmeldenummer: 05005203.4
(22) Anmeldetag: 10.03.2005
(51) Int. Cl.: A61K 38/36, A61L 27/34, A61L 27/38, A61L 27/56

(54) **Knochenersatzmittel enthaltend Faktor XIII**

(30) Priorität: 30.03.2004 DE 102004016065
(71) Anmelder: ZLB Behring GmbH und Mathys Medizinaltechnik AG, 35041 Marburg (DE)
(72) Erfinder: Wrabetz, Erhardt, 65239 Hochheim (DE); Rode, Michael, 61440 Oberursel (DE); Stoll, Thierry, 2572 Sutz (CH); Becker, Stephan, 1130 Wien (AT); Wilke, Ingo, 37133 Friedland (DE)
(74) Vertreter: Pfeil, Hugo

(57) **Zusammenfassung**

Es wird ein Knochenersatzmittel beschrieben, das aus einem metallporösen oder einem biokompatiblen, offenporigen Material besteht, welches ganz oder teilweise mit einer den Faktor XIII enthaltenden Lösung imprägniert oder zumindest ein Teil seiner Poren mit einer den Faktor XIII enthaltenden Lösung gefüllt ist.

## Beschreibung

Die Erfindung betrifft ein Knochenersatzmittel, bestehend aus einem metallporösen oder einem biokompatiblen, offenporigen Material.

Aus der internationalen Patentanmeldung WO 02/15950 ist bereits ein Verfahren zur Herstellung eines Knochenersatzmaterials bekannt, bei dem ein biokompatibler, offenporiger Körper einem Vakuum ausgesetzt wird und osteoinduktive und/oder osteogene Substanzen in fließfähiger Form mittels des in den Poren des Körper erzeugten Vakuums in diese Poren hineingesaugt werden. Damit ist es möglich, ein Knochenersatzmaterial herzustellen, welches in den Poren des biokompatiblen Körpers osteoinduktive und/oder osteogene Substanzen enthält, welche als Netzstruktur für neue, in den porösen Körper einzuwachsende Knochenzellen dienen.

Durch dieses bekannte Verfahren werden die bisher angewendeten Methoden zur Knochenneubildung bereits erheblich verbessert. Es besteht jedoch der Wunsch, die Knochenneubildung noch weiter zu vereinfachen und zu beschleunigen.

Es ist außerdem bereits bekannt, dass der Blutgerinnungsfaktor XIII bei systemischer Applikation neben seiner wundheilenden Wirkung auch positive Effekte auf die frühe Kallusbildungs- und die späte Kallusreifungsphase ausübt. Die mechanische Belastbarkeit des Kallus wird dabei signifikant erhöht. Die Ergebnisse der Knochenheilung stehen in enger Korrelation zur gewählten Dosis. Als optimale Dosis wurden 10 und 50 E./kg ermittelt. Die positiven Ergebnisse sind darauf zurückzuführen, dass Faktor XIII einerseits die Kallusbildung vermutlich durch die mitogene Wirkung auf die Osteoblasten quantitativ anregt, andererseits kommt es durch die raschere Fibrinvernetzung im Hämatom zu einer schnelleren Kallusbildung. Die erhöhte Vernetzungsgeschwindigkeit des Fibrins kann früher günstige Bedingungen für die Knochenneubildung im Kallus schaffen. Somit reduziert eine Faktor XIII-Gabe die sehr langen Behandlungszeiten bei Störungen der Kallusbildung und - reifung, bspw. in Fällen von Pseudoarthrosen oder Kallusdistraktionen. Darüber hinaus kann auch die Komplikationsrate vermindert werden. Die Imprägnierung eines Knochenersatzmittels mit Faktor XIII und die sich daraus ergebenden besonderen Vorteile für die Knochenregeneration sind dagegen bisher noch nicht beschrieben worden.

Durch zahlreiche Versuche wissenschaftlicher Arbeitsgruppen ist bestätigt worden, dass Faktor XIII die Knochenheilung beschleunigen und verbessern kann. Es stellte sich deshalb die Frage, ob durch eine Kombination des aus der Internationalen Patentanmeldung WO 02/15950 bekannten Verfahren zur Herstellung eines Knochenersatzmaterials mit der gleichzeitigen Applikation von Faktor XIII eine Beschleunigung der Knochenregeneration zu erzielen ist. Wichtige Behandlungsmethoden bestehen dabei einerseits in dem Einsetzen eines Knochenimplantats als auch in der in vivo- oder in vitro-Behandlung des Knochenmaterials.

Zu den Grundvoraussetzungen für das Gelingen einer Implantatverankerung mit poröser Oberfläche zählen die Anwendung eines Werkstoffes mit hoher Biokompatibilität sowie die Optimierung der lokalen Grenzflächenvoraussetzungen in Form von adäquater Porengröße, Ausschluss von Relativbewegungen an der Implantatknochengrenzfläche und der direkte Implantat-Knochenkontakt. Bisher sind überwiegend metallische Werkstoffe von den Implantatherstellern verwendet worden, wobei Porengrößen zwischen 100 µ und 500 µ angewendet wurden. Soweit bei diesen Versuchen der Effekt der systemischen Gabe von Faktor XIII-Konzentrat sowie des rekombinanten Faktor XIII auf das Knocheneinwachsverhalten und die Verankerungsfestigkeit von metallporösen Oberflächenimplantaten untersucht werden konnte, war zwar ein positiver Effekt erkennbar, der sich jedoch nicht als signifikant darstellen ließ. Ob das Knocheneinwachsverhalten und die Verankerungsfestigkeit einerseits und die Neubildung von Knochenmaterial auf einem biokompatiblen, offenporigen Knochenmaterial andererseits zufrieden stellende Ergebnisse liefern würde, konnte den bisher bekannten Versuchsergebnissen nicht entnommen werden. Beschichtungen von metallporösem oder biokompatiblem, offenporigen Materialien mit Faktor XIII sind bisher noch nicht als Knochenersatzmaterial eingesetzt worden.

Gegenstand der Erfindung ist deshalb ein Knochenersatzmittel, bestehend aus einem metallporösen oder einem biokompatiblen, offenporigen Material, das ganz oder teilweise mit einer den Faktor XIII enthaltenden Lösung imprägniert oder zumindest ein Teil seiner Poren mit einer den Faktor XIII enthaltenden Lösung gefüllt ist.

Vorzugsweise soll das Knochenmaterial aus einer biokompatiblen, offenporigen Substanz bestehen, die mindestens teilweise bioresorbierbar ist. Ganz besonders haben sich Hydroxylapatit und Tricalciumphosphat hierfür als geeignet erwiesen. Es kann aber auch körpereigene Knochensubstanz oder Tricorallit eingesetzt werden. Metallporöse Materialien haben den Vorzug, aufgrund ihrer großen Festigkeit, dem Knochen eine hohe Stabilität zu verleihen.

Das Knochenersatzmaterial soll eine Porosität von mindestens 25%, vorzugsweise von mind. 35% aufweisen, wobei über 50% der Poren einen Durchmesser im Bereich von 200 bis 500 Mikron aufweisen sollten Besonders geeignet ist ein Bioersatzmaterial, bei dem die Verbindungskanäle zwischen den einzelnen Poren einen Durchmesser im Bereich von 10 bis 300 Mikron, vorzugsweise 200 bis 400 Mikron aufweisen.

Der Faktor XIII kann dem Knochenersatzmaterial auf ganz unterschiedliche Weise zugegeben werden. Gut geeignet ist auch die Verwendung von Faktor XIII in Form von Mikrokapseln mit protrahierter Wirkstofffreigabe, bei denen die Kapselwand aus biologisch abbaubaren, synthetischen Materialien, z.B. Polymilchsäure, oder Proteinen besteht.

Der große Vorteil des erfindungsgemäßen Knochenersatzmittels gegenüber dem Einsatz eines Faktor XIII-freien, metallporösen oder resorbierbaren, offenporigen Material, bei dem der Faktor XIII dem Patienten in parenteraler Form verabreicht wird, besteht darin, dass bei dem erfindungsgemäßen Knochenersatzmaterial besonders hohe Faktor XIII Konzentrationen am zu behandelnden Knochen sichergestellt werden. Dadurch werden besonders gute Voraussetzungen für eine schnelle und wirksame Knochenneubildung geschaffen.

Eine weitere Beschleunigung der Knochenneubildung lässt sich erreichen, wenn dem erfindungsgemäßen Knochenersatzmittel noch zusätzliche Zellen aus dem autologen Knochenmark oder andere Knochen bildende Zellen des Patienten oder auch aus seinem Periost gewonnene Zellen zugefügt werden.

Der Erfolg der Knochenneubildung kann auch durch die Anwendung von osteo- induktiven und/oder osteogenen Substanzen gefördert werden, die zusätzlich zum Faktor XIII und den aus dem autologen Knochenmark gewonnenen Zellen eingesetzt werden. Hierfür kommen insbesondere die folgenden Substanzen in Betracht, die vorzugsweise in Form einer Suspension verabreicht werden:
a) synthetische Wachstumsfaktoren,
b) rekombinante Wachstumsfaktoren, vorzugsweise o-Wachstumsfaktor (TGF- □) oder FGF-2 (Fibroblast Growth Factor);
c) natürliche oder synthetische Peptide;
d) blutplättchenabgeleiteter Wachstumsfaktor (PDGF);
e) insulinartiger Wachstumsfaktor (IGF);
f) Fibrin als Endprodukt der Blutgerinnung,
g) synthetisches Fibrin oder
h) Proteine der bone morphogenetic protein-Familie (BMP).

Die Suspension der osteoinduktiven oder osteogenen Substanzen kann in einer körperverträglichen Suspension, vorzugsweise in einer wässrigen Suspension verabreicht werden.

Das erfindungsgemäße Knochenersatzmittel kann auf verschiedene Weise hergestellt werden. Im allgemeinen wird man das metallporöse oder biokompatible, offenporige Knochenmaterial unmittelbar vor der Anwendung mit einer den Faktor XIII enthaltenden Lösung imprägnieren, indem man durch Anlegen eines Vakuums die Lösung in die Poren des Materials einsaugt. Es ist jedoch auch möglich, das Knochenmaterial mit einer den Faktor XIII enthaltenden Lösung zu beschichten oder zu vermischen. In gleicher Weise können die vorstehend genannten Zellen aus autologem Knochenmark oder die aus dem Periost gewonnenen Zellen sowie die vorstehend genannten osteoinduktiven und/oder osteogenen Substanzen mit dem Knochenersatzmittel in Kontakt gebracht werden. Entscheidend ist lediglich, dass eine intensive Benetzung der äußeren und inneren Oberfläche des porösen Knochenersatzmaterials erreicht wird.

Die in den verletzten Knochen zusammen mit dem metallporösen oder dem biokompatiblen, offenporigen Material einzuführende Menge des Faktor XIII betrug bisher im allgemeinen 10 bis 50 Einheiten/kg Körpergewicht bei intravenöser Applikation. Wird das Knochenersatzmaterial jedoch erfindungsgemäß mit einer Faktor XIII-Lösung imprägniert oder seine Poren zumindest teilweise mit einer den Faktor XIII enthaltenden Lösung gefüllt, dann reichen 0,05 bis 10 Einheiten Faktor XIII/kg Körpergewicht aus.

Bei Anwendung des beschriebenen Verfahrens beobachtet man schon nach wenigen Tagen die Ausbildung eines körnigen, faserarmen, zell- und gefäßreichen Bindegewebes, des Granulationsgewebes. In diesem Gewebe beginnen dann diverse Zellen mit dem Aufbau einer knorpeligen Matrix. Dieser Prozess schreitet fort, bis das ganze Granulationsgewebe durch Knorpel ersetzt und später kalzifiziert wird.

Die Anwendung des erfindungsgemäß mit Faktor XIII angereicherten, metallporösen oder biokompatiblen, offenporigen Knochenersatzmaterial beschleunigt die Knochenneubildung ganz erheblich. In vivo kann die Knochenneubildung hiermit um bis zu 40% verkürzt werden.

## Patentansprüche

1. Knochenersatzmittel bestehend aus einem metallporösen oder biokompatiblen, offenporigen Material, **dadurch gekennzeichnet, dass** es ganz oder teilweise mit einer den Faktor XIII enthaltenden Lösung imprägniert oder zumindest ein Teil seiner Poren mit einer den Faktor XIII enthaltenden Lösung gefüllt ist.

2. Knochenersatzmittel nach Anspruch 1, **dadurch gekennzeichnet, dass** das biokompatible, offenporige Material mindestens teilweise bioresorbierbar ist und vorzugsweise aus Hydroxylapatit oder Tricalciumphosphat besteht.

3. Knochenersatzmittel nach Anspruch 1, **dadurch gekennzeichnet, dass** seine Porosität mindestens 25%, vorzugsweise mindestens 35% beträgt.

4. Knochenersatzmittel nach Anspruch 1, **dadurch gekennzeichnet, dass** über 50% der Poren einen Durchmesser im Bereich von 200 bis 500 Mikron aufweisen.

5. Knochenersatzmittel nach Anspruch 1, **dadurch gekennzeichnet, dass** die Verbindungskanäle zwischen den einzelnen Poren einen Durchmesser im Bereich von 10 bis 300 Mikron, vorzugsweise 200 bis 400 Mikron aufweisen.

6. Knochenersatzmittel nach Anspruch 1, **dadurch gekennzeichnet, dass** es zusätzlich Zellen aus autologem Knochenmark enthält.

7. Knochenersatzmittel nach Anspruch 1, **dadurch gekennzeichnet, dass** es außerdem aus dem Periost gewonnene Zellen enthält.
